# EUROPEAN PATENT APPLICATION

(11) **EP 3 824 833 A1**
(43) Date of publication of application: **26.05.2021**
(21) Application number: 20207458.9
(22) Date of filing: 13.11.2020
(51) Int. Cl.: A61B 17/88, A61B 17/70

(54) **EXPANDABLE VERTEBRAL DEVICE WITH TRIANGULAR CONNECTION BEAM**

(30) Priority: 15.11.2019 TW 108141587
(71) Applicant: BioLife Medical Device Inc., Hsinchu City 300 (TW)
(72) Inventor: CHEN, Li-Sen, 300 Hsinchu City (TW); CHAO, Che-Yang, 300 Hsinchu City (TW); KAO, Li-Hwan, 300 Hsinchu City (TW)
(74) Representative: Viering, Jentschura & Partner mbB Patent- und Rechtsanwälte

(57) **Abstract**

An expandable vertebral device (100) with triangular connection beam includes: a rotatable center shaft (101); a fixed part (103), wherein the rotatable center shaft penetrates through the fixed part and is rotatable relative to the fixed part; a movable slide (104), surrounding a portion of the rotatable center shaft, and is movable along an axial direction of the rotatable center shaft in response to a rotation of the rotatable center shaft; at least one first connection beam (105), which is rotatably connected to the fixed part; at least one second connection beam (106), which is rotatably connected to the movable slide, and is rotatably connected to the corresponding first connection beam; whereby when the rotatable center shaft rotates within the fixed part so that the movable slide moves away from the fixed part, the first and second connection beams are expanded to restore a vertebra to its original shape.

## Description

### CROSS REFERENCE

The present invention claims priority to TW 108141587 filed on November 15, 2019.

### BACKGROUND OF THE INVENTION

### Field of Invention

The present invention relates to an expandable vertebral device with triangular connection beam; particularly, it relates to such an expandable vertebral device which, when inserted into a human body, can expand itself by means of the triangular connection beams, so as to restore a vertebra to its original shape.

### Description of Related Art

When a human spine (e.g. cervical vertebrae, thoracic vertebrae, lumbar vertebrae, etc.) suffers a compression fracture, or collapses due to poor bone mass density, a vertebral device which is inserted into the spine can help expand the space to restore the spine to a better condition. US 7,846,206 B2, US 9,414,933 B2, and TW 1589266 disclose different types of vertebral devices.

In comparison with the prior arts, the present invention uses a different mechanical structure with triangular connection beams, whereby the number of required components is reduced and the vertebral device has a better mechanical strength to resist deformation.

### SUMMARY OF THE INVENTION

From one perspective, the present invention provides an expandable vertebral device with triangular connection beam, comprising: a rotatable center shaft; a fixed part, wherein the rotatable center shaft penetrates through the fixed part and is rotatable relative to the fixed part; a movable slide, surrounding a portion of the rotatable center shaft, is movable along an axial direction of the rotatable center shaft in response to a rotation of the rotatable center shaft; at least one first connection beam, which is pivotally connected to the fixed part; at least one second connection beam, which is pivotally connected to the movable slide, and is pivotally connected to the corresponding first connection beam; whereby when the rotatable center shaft rotates within the fixed part to drive the movable slide to move relative to the fixed part, the first and second connection beams are expanded toward a radial direction of the rotatable center shaft.

In one embodiment, the first connection beam is longer than the second connection beam.

In one embodiment, an outer surface of the rotatable center shaft and an inner surface of the movable slide are threaded.

In one embodiment, wherein the outer surface of the rotatable center shaft has an edge which is configured to restrain the fixed part.

In one embodiment, the expandable vertebral device further comprises a nut which is fixed to one end of the rotatable center shaft.

In one embodiment, the rotatable center shaft is hollow inside and is provided with an inlet and at least one outlet.

In one embodiment, the rotatable center shaft is provided with at least one outlet at a radial surface of the rotatable center shaft.

In one embodiment, the rotatable center shaft has an operable end that is configured to be driven by a corresponding tool to rotate the rotatable center shaft.

In one embodiment, the operable end has a hexagonal shape.

The objectives, technical details, features, and effects of the present invention will be better understood with regard to the detailed description of the embodiments below, with reference to the attached drawings.

### BRIEF DESCRIPTION OF THE DRAWINGS

Fig. 1 shows an expandable vertebral device with triangular connection beam according to one embodiment of the present invention, in closed state.
Fig. 2 shows the expandable vertebral device of Fig. 1, in its expanded state.
Fig. 3 shows an exploded view of the expandable vertebral device in Fig. 1.
Fig. 4 shows an expandable vertebral device with triangular connection beam according to another embodiment of the present invention, in expanded state.
Fig. 5 shows the first and second connection beams of the expandable vertebral device in Fig. 4.
Fig. 6 shows an expandable vertebral device with triangular connection beam according to another embodiment of the present invention, in its expanded state.
Fig. 7 shows the expandable vertebral device of Fig. 6, in its closed state.

### DESCRIPTION OF THE PREFERRED EMBODIMENTS

The drawings as referred to throughout the description of the present invention are for illustration only, to show the interrelations between the components, but not drawn according to actual scale of shapes and sizes.

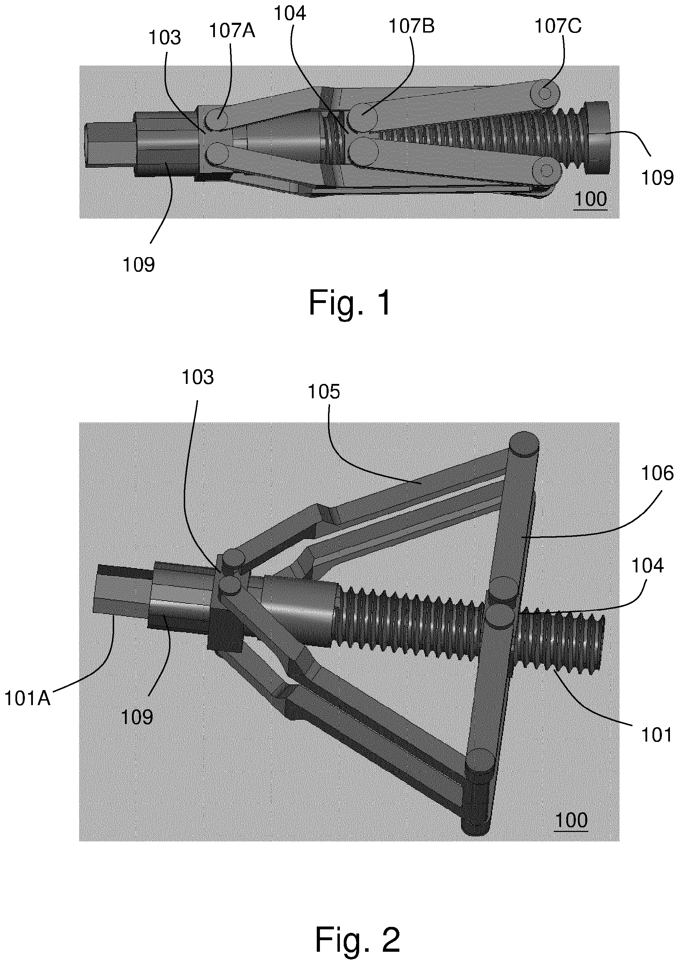

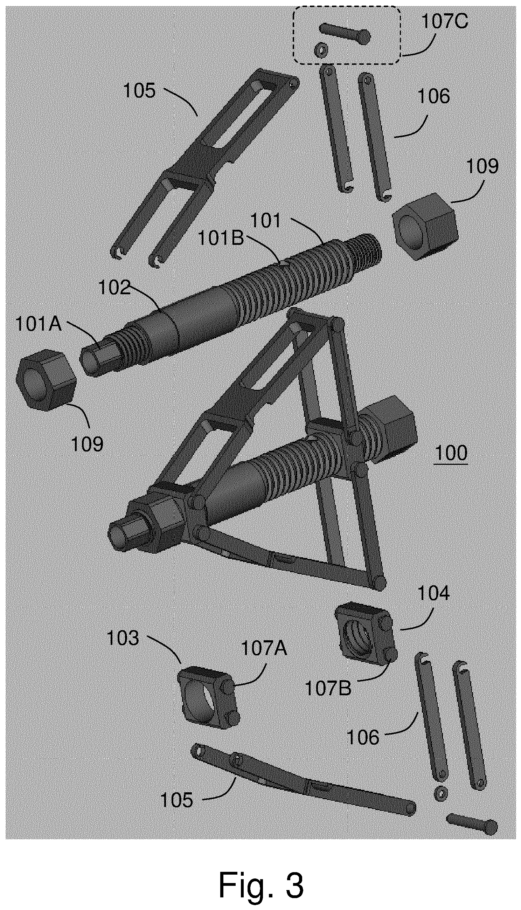

Please refer to Figs. 1-3, which show an expandable vertebral device with triangular connection beam according to an embodiment of the present invention (i.e. expandable vertebral device 100), in its closed state (Fig. 1), its expanded state (Fig. 2) and an exploded view (Fig. 3, which shows the expandable vertebral device in its assembled state and the components in its disassembled state), respectively.

The expandable vertebral device 100 includes a rotatable center shaft 101, a fixed part 103, a movable slide 104, at least one first connection beam 105, and at least one second connection beam 106. The rotatable center shaft 101 penetrates through the fixed part 103 and is rotatable relative to the fixed part 103; the movable slide 104 surrounds a portion of the rotatable center shaft 101, and is movable along an axial direction of the rotatable center shaft 101 in response to a rotation of the rotatable center shaft 101; the at least one first connection beam 105 is pivotally connected to the fixed part 103 (in this embodiment, for example through a connection member 107A which allows relative pivoting of the first connection beam 105); each first connection beam 105 is pivotally connected to a corresponding second connection beam 106 (in this embodiment, for example through a connection member 107C which allows relative pivoting of the second connection beam 106), and the at least one second connection beam 106 is pivotally connected to the movable slide 104 (in this embodiment, for example through a connection member 107B which allows relative pivoting of the second connection beam 106) . Each of the connection members 107A, 107B and 107C can be a stand-alone component by itself and assembled to the fixed part 103 or movable slide 104, or be manufactured in one piece with one of the fixed part 103, the first connection beam 105, the second connection beam 106, and the movable slide 104. More specifically, the connection member 107A can be manufactured in one piece with the fixed part 103 or the first connection beam 105; the connection member 107B can be manufactured in one piece with the movable slide 104 or the second connection beam 106; the connection member 107C can be manufactured in one piece with the first connection beam 105 or the second connection beam 106.

In one embodiment, one or two ends of the rotatable center shaft 101 can be fitted into a nut or nuts 109, to prevent the fixed part 103 from detaching from the rotatable center shaft 101, and/or to prevent the movable slide 104 from detaching from the rotatable center shaft 101. However, if this is not a concern, then the nuts 109 can be omitted. Also please note that to avoid the detaching of the fixed part 103 or the movable slide 104 by means of nuts 109 is only an illustrative example, and there can be other ways to block or restrict the movable range of the movable slide 104, which should all be regarded as in the disclosure of this specification. In one embodiment, as shown in Fig. 3, the rotatable center shaft 101 is provided with a peripheral edge 102, so that the nut 109 at the left side, in the above drawing, can press the fixed part 103 close to the edge 102 to hold the fixed part 103 at a predetermined position by restraining the fixed part 103 with the edge 102. In one embodiment, the inner surface of the fixed part 103 and the corresponding predetermined portion of the outer surface of the rotatable center shaft 101 are smooth (i.e. without threads), so that the relative rotation between the rotatable center shaft 101 and the fixed part 103 is easier.

Please refer to Fig. 1 in conjunction with Fig. 2. Fig. 1 shows the expandable vertebral device according to the present invention in its closed state, while Fig. 2 shows the expandable vertebral device of Fig. 1 in its expanded state. The right side of the expandable vertebral device, in the drawing of the disclosure in this specification, is the front end and the left side of the expandable vertebral device, in the drawing of the disclosure in this specification, is the rear end; when a doctor operates to insert the expandable vertebral device into a human body, the right side (front end) enters the human body first (as compared the left side, rear end) .

The left side (rear end) of the rotatable center shaft 101 has an operable end 101A; the doctor can use a corresponding tool to rotate the operable end 101A, so that the rotatable center shaft 101 rotates relative to the fixed part 103. In the shown embodiment, the operable end 101A has a hexagonal shape and can be rotated by a complemental hexagonal driver; however, the tooling driving the rotatable center shaft 101 can be varied according to actual needs. The operable end 101A can be in any shape or form that can be driven to rotate by a corresponding/complementing tool(s).

When the rotatable center shaft 101 rotates, the movable slider 104 is driven to move relative to the fixed part 103, the distance between the fixed part 103 and movable slide 104 changes. When the relative rotation between the rotatable center shaft 101 and the fixed part 103 causes the movable slide 104 to move away from the fixed part 103, the angle (s) between the first connection beams 105 (plural in this embodiment) and the second connection beams 106 (plural in tis embodiment) are changed to make the device become expanded, forming upward and downward triangle structures to expand the space occupied by the device, therefore restoring the spine to an improved condition. In one embodiment, in order to control the movement range of the movable slide 104, on one hand, along the axial direction of the rotatable center shaft 101 in response to the rotation of the rotatable center shaft 101, and on the other hand, for maintaining the movable slide 104 at a predetermined stable position once the movable slide 104 reaches a desired position, the outer surface of the rotatable center shaft 101 (where the movable slide 104 may pass through) and the inner surface of the movable slide 104 are preferably provided with threads. In addition, in one embodiment, as shown in the figures, the first connection beam 105 can be relatively longer than the second connection beam 106, whereby in the closed state, the first connection beam 105 and the second connection beam 106 are rather close to the rotatable center shaft 101, so that the front end of the expandable vertebral device in the drawing(s) of this specification has a narrower profile, so that during the operation, the device can be inserted into the human body with more ease. If the first connection beam 105 is relatively shorter and the second connection beam 106 is relatively longer or the first connection beam 105 and the second connection beam 106 are of the same size, then the front end of expandable vertebral device in the drawing (s) of this specification will have a larger profile, which is disadvantageous for insertion during the operation.

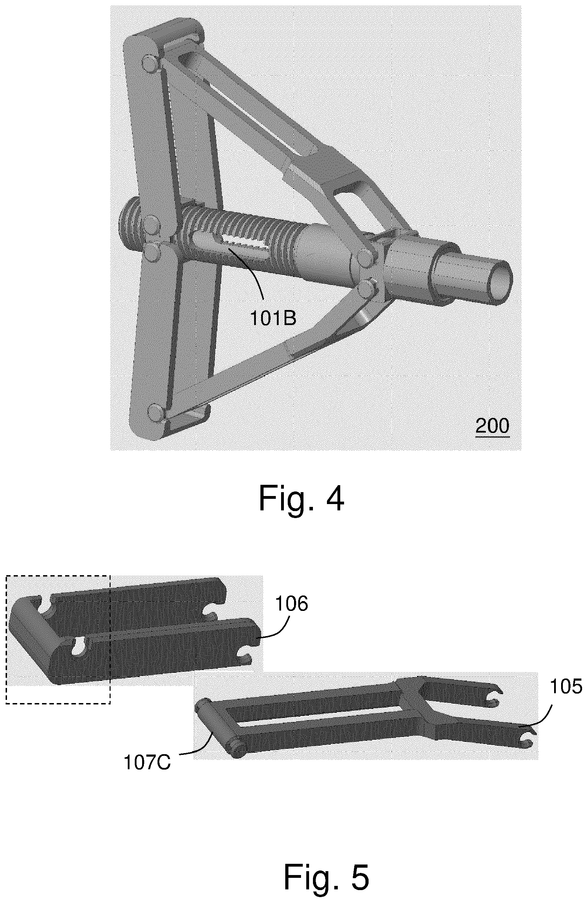

When the first connection beam 105 and the second connection beam 106 are expanded at its closed state, the first connection beam 105 and the second connection beam 106 not only pivot relative to each other, but also pivot relative to the fixed part 103 and the movable slide 104 respectively. Therefore, these components need to be connected with one another in a way which allows for relative pivoting. However, there are many ways to allow relative pivoting between the above said components, and the scope of the disclosures should include such variations. For example, as shown by the connecting member 107C in Fig. 3, a rod/pin through a corresponding hole and engaging with a complemental nut is one way to allow relative rotation. In another example, please refer to Fig. 4 which shows an expandable vertebral device 200 according to another embodiment of the present invention in its expanded state, and Fig. 5 which shows the first connection beam 105 and the second connection beam 106 in the expandable vertebral device 200 of Fig. 4, the first connection beam 105 and the second connection beam 106 can be pivotally connected with each other by another way. In this embodiment, the connecting member 107C is manufactured in one piece with the first connection beam 105, and the second connection beam 106 is provided with a complemental notch (referring to the dashed-line block) ; this is an example of a variation to allow relative pivoting. Certainly, it is equivalent to manufacture the connecting member 107C in one piece with the second connection beam 106 and to provide a complemental notch in the first connection beam 105. The above examples show that there can be many variations to allow for relative pivoting, and the scope of the disclosure should include such variations.

In one embodiment, the rotatable center shaft 101 comprises a hollow interior, and the rotatable center shaft is provided with an inlet and at least one outlet, so that when the expandable vertebral device is inserted into the human body, a filler such as bone cement or bone graft can be injected from the inlet, through the hollow interior, to outflow from the outlet into the vertebrae. Please refer to Fig. 3, in one embodiment, the filler injected form the inlet at the rear end (left side) of the rotatable center shaft 101, in addition to flowing through the hollow interior of the rotatable center shaft 101 and flowing out from the outlet at the right side of the rotatable center shaft 101, said fillers can also flow out in the radial direction from the outlets (e.g. outlets 101B as shown in Figs. 3 and 4) provided on the radial surface of the rotatable center shaft 101, so the device can provide multiple channels for releasing said fillers into the vertebrae. The number, size and shape of the outlets 101B shown in the figures are illustrative examples; variations in number, size and shape of the outlets should also regarded as disclosed within this specification.

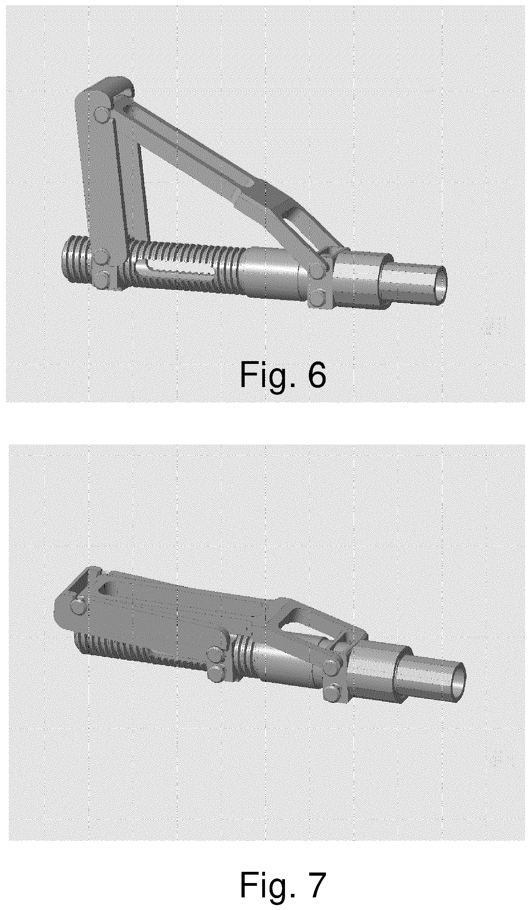

Please refer to Figs. 6 and 7, wherein Fig. 6 shows an expandable vertebral device according to another embodiment of the present invention in its expanded state, and Fig. 7 shows the expandable vertebral device of Fig. 6 in its closed state. This embodiment shows that: the first connection beam 105 and the second connection beam 106 can be provided at only one lateral side (as shown in the above said drawings of this embodiment) of the rotatable center shaft 101, instead of opposing two sides as the other drawings in this specification. Figs. 6 and 7 show that the first connection beam 105 and the second connection beam 106 can be provided at one side, whereas Figs. 1-5 show that the first connection beam 105 and the second connection beam 106 can be provided at two sides. However, under the spirit of the present invention, there can be three or more pairs of the first connection beam 105 and the second connection beam 106, which can be located in symmetrical or non-symmetrical arrangement around the rotatable center shaft 101.

The present invention has been described in considerable detail with reference to certain preferred embodiments thereof. It should be understood that the description is for illustrative purpose, not for limiting the scope of the application of the present invention. An embodiment or a claim of the present invention does not need to be regarded as having to achieve all the objectives or advantages of the present invention. The title and abstract are provided for assisting searches but not for limiting the scope of the present invention. Those skilled in this art can readily conceive variations and modifications within the spirit of the disclosure in this specification, which should therefore be included in the scope of the following claim(s) made by the inventors. For example, as long as the primary objective is attained, other extra components can be added, or, components that are given separate reference numbers and described as separate items can be combined into one piece. As an example, the second connection beams 106 shown in the upper part and lower part of Fig. 3 can be combined into one piece. As another example, the numbers, sizes and shapes of the first connection beam 105 and the second connection beam 106 can be modified. It is not limited for each of the embodiments described hereinbefore to be used alone; under the spirit of the present invention, two or more of the embodiments described hereinbefore can be used in combination. For example, two or more of the embodiments can be used together, or, a part of one embodiment can be used to replace a corresponding part of another embodiment. In view of the foregoing, the spirit of the present invention should cover all such and other modifications and variations, which should be interpreted to fall within the scope of the following claims and their equivalents.

## Claims

1. An expandable vertebral device with triangular connection beam, comprising:
a rotatable center shaft;
a fixed part, wherein the rotatable center shaft penetrates through the fixed part and is rotatable relative to the fixed part;
a movable slide, surrounding a portion of the rotatable center shaft, and is movable along an axial direction of the rotatable center shaft in response to a rotation of the rotatable center shaft;
at least one first connection beam, which is pivotally connected to the fixed part;
at least one second connection beam, which is pivotally connected to the movable slide, and is pivotally connected to the corresponding first connection beam;
whereby when the rotatable center shaft rotates within the fixed part to drive the movable slide to move relative to the fixed part, the first and second connection beams are expanded toward a radial direction of the rotatable center shaft.

2. The expandable vertebral device of claim 1, wherein the first connection beam is longer than the second connection beam.

3. The expandable vertebral device of claim 1, wherein an outer surface of the rotatable center shaft and an inner surface of the movable slide are threaded.

4. The expandable vertebral device of claim 1, wherein the outer surface of the rotatable center shaft has an edge which is configured to restrain the fixed part in a predetermined location.

5. The expandable vertebral device of claim 1, further comprising a nut which is fixed to at least one end of the rotatable center shaft.

6. The expandable vertebral device of claim 1, wherein the rotatable center shaft comprises a hollow interior and is provided with an inlet and at least one outlet for the injection of a filler into a human body.

7. The expandable vertebral device of claim 6, wherein the rotatable center shaft is provided with at least one outlet on a radial surface of the rotatable center shaft.

8. The expandable vertebral device of claim 1, wherein the rotatable center shaft has an end which is an operable end that is configured to be driven by a corresponding tool to rotate the rotatable center shaft.

9. The expandable vertebral device of claim 8, wherein the operable end has a hexagonal shape.
